# EUROPEAN PATENT SPECIFICATION

(11) **EP 1 794 289 B1**
(45) Date of publication and mention of the grant of the patent: **22.09.2010**
(21) Application number: 05794666.7
(22) Date of filing: 30.09.2005
(51) Int. Cl.: C12N 5/16

(54) **METHOD FOR EX-VIVO PURGING IN AUTOLOGOUS TRANSPLANTATION USING SOLUBLE FAS-L MOLECULES**
EX-VIVO-SPÜLVERFAHREN FÜR AUTOLOGE TRANSPLANTATIONEN MITTELS LÖSLICHER FASL-MOLEKÜLE
PROCEDE DE PURGE EX VIVO EN AUTOGREFFE AU MOYEN DE MOLECULES FASL SOLUBLES

(30) Priority: 01.10.2004 US 615084 P
(43) Date of publication of application: 13.06.2007
(73) Proprietor: Topotarget Switzerland SA, 1004 Lausanne (CH)
(72) Inventor: DUPUIS, Marc, CH-1807 BLONAY (CH); GREANEY, Peter, CH-1034 BOUSSENS (CH); DUCHOSAL, Michel, CH-1093 LA CONVERSION (CH)
(74) Representative: Tetaz, Franck Claude Edouard
(86) International application number: PCT/EP2005/054950
(87) International publication number: WO 2006/037762

(56) References cited:
- ANONYMOUS: "MegaFasL as a medical device for ex vivo purging in autologous transplantation" INTERNET ARTICLE, 2003, XP002357194 Retrieved from the Internet: URL:https://wwwdbpub.unil.ch/admin/?MIval= RcIRRProj&AnRech=2004&UrId=106&LanCode=37> [retrieved on 2005-11-30]
- GREANY, P, ET AL: "A Fas agonist induces high levels of apoptosis in haematological malignancies" LEUKEMIA RESEARCH, 20 September 2005 (2005-09-20), pages 1-12, XP002357195 Retrieved from the Internet: URL:http://www.sciencedirect.com/science?_ ob=MImg&_imagekey=B6T98-4H57JPB-2-C&_cdi=5 108&_user=987766&_orig=search&_coverDate=0 9%2F21%2F2005&_qd=1&_sk=999999999&view=c&w chp=dGLzVzz-zSkzk&md5=d9153e90dda56984751a 82e665ffad11&ie=/sdarticle.pdf>
- ZHANG YICHENG ET AL: "Purging of contaminating breast cancer cells from hematopoietic progenitor cell preparations using activation enhanced cell death" BREAST CANCER RESEARCH AND TREATMENT, vol. 72, no. 3, April 2002 (2002-04), pages 265-278, XP002357196 ISSN: 0167-6806
- HOLLER NILS ET AL: "Two adjacent trimeric Fas ligands are required for Fas signaling and formation of a death-inducing signaling complex" MOLECULAR AND CELLULAR BIOLOGY, AMERICAN SOCIETY FOR MICROBIOLOGY, WASHINGTON, US, vol. 23, no. 4, February 2003 (2003-02), pages 1428-1440, XP002258597 ISSN: 0270-7306
- CARLO-STELLA C ET AL: "Effects of the tyrosine kinase inhibitor AG957 and an Anti-Fas receptor antibody on CD34(+) chronic myelogenous leukemia progenitor cells." BLOOD. 1 JUN 1999, vol. 93, no. 11, 1 June 1999 (1999-06-01), pages 3973-3982, XP002357197 ISSN: 0006-4971
- DEBATIN KLAUS-MICHAEL ET AL: "Death receptors in chemotherapy and cancer." ONCOGENE. 12 APR 2004, vol. 23, no. 16, 12 April 2004 (2004-04-12), pages 2950-2966, XP002357198 ISSN: 0950-9232
- LI Q ET AL: "Qualitative and quantitative characterization of Fas (CD95) expression and its role in primary human acute leukemia cells." LEUKEMIA RESEARCH. MAY 2000, vol. 24, no. 5, May 2000 (2000-05), pages 437-444, XP002357199 ISSN: 0145-2126

## Description

The present invention concerns a new method for ex-vivo purging of cells in autologous transplantation according to claim 1.

Autologous stem cell transplantation (ASCT) following high-dose chemotherapy with or without radiotherapy has become the standard therapy for the majority of patients with large-cell lymphomas, multiple myeloma, and refractory/recidivating Hodgkin's disease. Such therapy is nowadays also contemplated for selected patients with low-grade lymphomas (chronic lymphocytic leukemia, follicular lymphoma, mantle cell lymphoma) and for patients with acute myeloid leukemia (AML). Current treatments for cell purging include chemotherapy and antibody cocktails. These treatments are often toxic on stem cells and not efficient in eliminating cancer cells. Thus, there is an unmet medical need for cell purging in ASCT which this project will address.

As of today, more than 670,000 Americans have blood cancer. In the US, every five minutes, someone is diagnosed with blood cancer, which means that more than 106,000 new cases are expected to be diagnosed this year. Every nine minutes, someone dies from blood cancer in the US, which represents an estimated 58,000 deaths (in 2003). Although it has been estimated that more than 30% of all autografts are contaminated with tumor cells, ASCT following high dose chemotherapy has gained extensive application as a therapeutic modality in many types of malignancies. As of today, ASCT largely surpass the number of allotransplantations.

In 2002, 10,500 ASCT were performed in the US, which brings to an incidence in the general population of 3.7/100,000. In terms of comparison, in Europe (EU12), 11,500 ASCT were performed in 2001 with an incidence of 3.1/100,000. Therefore, these incidence numbers indicate that at least 20,000 ASCT will be performed between Europe and the United States in 2004. In the last decade, ASCT has experienced an enormous increase in activity. As an example, only 2,097 ASCT were recorded in Europe (EU35) in 1990. In 1999, the number of ASCT reached 12,841, representing a 600% increase in less than 10 years. Although the trend has indicated a slight regression in terms of the number of ASCT performed in 1999, recent data clearly show that the trend is positive since late 1999 with a constant progression in the number of ASCT performed.

Autologous hematopoietic stem cell transplantation (ASCT) is a procedure increasingly used for the treatment of malignancies including those of the hematolymphoid system (leukemias, lymphomas). In this procedure hematopoietic stem cells (HSC) are harvested from the patient prior to intensive chemotherapy, and such HSC are subsequently reinfused to rescue HSC function in the patient. The aim of autologous transplantation is to bypass the toxicity of chemotherapy to HSC, enabling higher dosis of chemotherapy to be administered, and higher efficiency of treatment towards tumoral cell eradication to be achieved. Such a procedure contemplates the reinfusion of an HSC graft potentially contaminated with tumoral cells. Indeed gene marking experiments have demonstrated the graft origin of lymphoid tumors/myeloid leukemia recidivating post ASCT, supporting the concept of graft cell purging. Additionally, molecular biology studies have demonstrated increased overall survival and disease-free survival in patients with no detectable tumoral cells following autologous HST. Indeed, purging has demonstrated its efficiency in increasing overall survival in patients with follicular lymphomas.

Purging procedures have included positive selection of CD34+ (a cell fraction enriched for HSC), negative selection of tumoral CD20+ cells, chemotherapy or administration of toxic molecules in the pouch. The drawbacks of such procedures is that they are "cumbersome and expensive" (Dalton WS and al., Hematology 2001, pp. 157), often drastically reduce the HSC activity of the graft, and that transplanted patients have higher incidence of infections, in relation with the delay in hematopoietic recovery associated with purging, and possibly with toxicity of the purging towards immune cells in the graft (Crippa and al. Bone Marrow transplant 8: 281, 2002). There is therefore an unmet medical need for ex vivo purging for tumor cells with a reasonable toxic profile in autologous HST.

### Current therapies

To minimize the risk of residual tumor cells in the graft, a variety of purging methods have been used. Widely cited purging methods include the use of:
□ *Ex vivo* chemotherapy (e.g. cyclophosphamide derivatives)
□ Tumor targeting monoclonal antibodies linked with toxins or selected on immunocolumns
□ Positive CD34+ selection.

The most recent developments concern photodynamic processes (e.g. CELMED company).

To date, no method mentioned above has proved to be satisfactory in completely depleting residual tumor cells while preserving hematopoietic and lymphoid activity of the autograft.

Because of its efficacy towards eradicating tumoral cells, and its low toxicity towards HSC and lymphoid cells, the use of soluble Fas-1 molecules, more particularly multimerized forms of soluble fractions of FasL such as the potent MegaFasL fulfils the criteria for being an ideal purging agent in ASCT.

The present invention concerns a new method for ex-vivo purging of cells in autologous transplantation according to claim 1.

In a preferred embodiment, the sample of harvested cells from the patient is maintained frozen prior to thawing and treatment immediately before re-infusion into the patient.

Such appropriate freezing procedures and medium are well known in the art. For instance, stem cells may be harvested and maintained in an anticoagulant solution such as ACD-A solution (sold by Laboratoriul Dr. G Bischeeel AG, Interlaken Switzerland). Said harvested cells may be then transferred for freezing into a specific freezing medium comprising DMSO and albumin such as CryoSure-DMSO solutions (sold by WAK-Chemie Medical GmbH, Steinbach, Germany) and Albumin ZLB 5%.

The final treatment concentration of soluble multimerized Fas-L molecules in the solution comprising the harvested cells is generally comprised between 1 and 400 ng/ml, preferably comprised between 10 and 50 ng/ml.

The harvested cells are preferably treated for a period comprised between 1 and 12 hours. In a specific embodiment, cells are treated for a period of time of about 5 hours.

After treatment with soluble multimerized Fas-L molecules, remaining living cells are preferably treated by extensive washing to eliminate remaining FAsL molecules in the medium. Physiologicaly acceptable saline solutions are generally used for such washing procedures. The presence of trace amounts of MegaFasL is checked by ELISA of the last wash supernatant, prior autologous transplantation.

Methods for purifying/washing the harvested cells after treatment according to the invention are well known in the art.

The soluble multimerized FasL molecules according to the invention comprise six globular soluble extracellular fractions of the Fas ligand bound to a multimerization moiety.

Multimerized FasL molecules may eventually aggregate to form higher degrees of multimerization, including dodecamer (2 hexamers) or octodecamers (3 hexamers).

In the methods of the present invention, the multimerized form of Fas ligand is a hexamer comprising six monomers, assembled together, each of the monomers comprising a polypeptide of formula (I):

H-L (I)

wherein
L comprises the extracellular domain of human FAS ligand (hFasL), comprising amino acids Glu 139 to Leu 281 of hFasL, and
H represents an N-terminal hexamerization moiety.

Hexamers according to the invention are either "true" hexamers, dimers of trimers or trimers of dimers. In the first case, H is a hexamerization polypeptide HP. In the latter cases, H comprises two moieties, a first moiety consisting of a dimerization polypeptide (DP) and a second moiety consisting of a trimerization polypeptide (TP).

The polypeptides according to the present invention comprise a polypeptide represented by one the following formulas (Ia), (Ib) and (Ic):

HP - L (Ia) ("true" hexamers),

DP-TP - L (Ib) (trimers of dimers),

and

TP-DP - L (Ic) (dimers of trimers)

wherein L, HP, DP and TP are defined above and below.

Examples of HP, TP and DP are well known in the art and comprise isolated peptide fragments of natural hexameric, trimeric or dimeric polypeptides, the said isolated fragments being responsible for the hexamerization, dimerization or trimerization of the said natural hexamers, dimers or trimers.

Such molecules are well known in the art and comprises polypeptides of the collectin family, such as the ACRP30 or ACRP30-like proteins (WO96/39429, WO 99/10492, WO 99/59618, WO 99/59619, WO 99/64629, WO 00/26363, WO 00/48625, WO 00/63376, WO 00/63377, WO 00/73446, WO 00/73448 or WO 01/32868), apM1 (Maeda et al., Biochem. Biophys. Res. Comm. 221: 286-9, 1996), Clq (Sellar et al., Biochem. J. 274: 481-90, 1991), or Clq like proteins (WO 01/02565), which proteins comprise "collagen domains" consisting in collagen repeats Gly-Xaa-Xaa'.

Other oligomerized polypeptides are known in the art, including polypeptides with a "coiled-coil" domains (Kammerer RA, Matrix Biol 1997 Mar;15(8-9):555-65; discussion 567-8; Lombardi & al., Biopolymers 1996;40(5):495-504; http://mdl.ipc.pku.edu.cn/scop/data/scop.1.008.001.html), like the Carilage Matrix Protein (CMP) (Beck & al., 1996, J. Mol. Biol., 256, 909-923), , or polypeptides with a dimerization domain, like polypeptides with a leucine zipper or osteoprotegerin (Yamaguchi & al., 1998).

According to a specific embodiment of the invention, HP comprises the hexamerization domains of A, B or C chains of polypeptides of the C1q family.

TP are known in the art and comprise the trimerization domains (C-terminal moiety) of CMP (i.e. GeneBank 115555, amino acids 451-493) or the trimerization domain of ACRP30 and ACRP30-like molecules. According to a preferred embodiment of the present invention, TP comprises a stretch of collagen repeats.

According to the invention, a "stretch of collagen repeats" consists in a series of adjacent collagen repeats of formula (II):

-(Gly-Xaa-Xaa')ₙ- (II)

wherein Xaa and Xaa' represents independently an amino acid residue, and n represents an integer from 10 to 40.

Xaa and Xaa' are preferably selected independently among natural amino acids such as Ala, Arg, Asn, Asp, Cys, Gln, Glu, Gly, His, Ile, Leu, Lys, Met, Phe, Pro, Ser, Thr, Trp, Tyr or Val.

Xaa preferably represents independently an amino acid residue selected among Ala, Arg, Asp, Glu, Gly, His, Ile, Leu, Met, Pro or Thr, more preferably Arg, Asp, Glu, Gly, His or Thr.

Xaa' preferably represents independently an amino acid residue selected among Ala, Asn, Asp, Glu, Leu, Lys, Phe, Pro, Thr or Val, more preferably Asp, Lys, Pro or Thr.

When Xaa' represents a Pro residue, the collagen repeat Gly-Xaa-Pro is designated to be a "perfect" collagen repeat, the other collagen repeats being designated as "imperfect".

According to a preferred embodiment of the invention, the stretch of collagen repeats comprises at least 1 perfect collagen repeat, more preferably at least 5 perfect collagen repeats.

According to a preferred embodiment of the invention, n is an integer from 15 to 35, more preferably from 20 to 30, most preferably 21, 22, 23 or 24.

According to the present invention, the stretch of collagen repeat may comprise up to three "non collagen residues" inserted between two adjacent collagen repeats. These "non collagen residues" consist in 1, 2 or 3 amino acid residues, provided that when the "non collagen residue" consists in 3 amino acids residues, the first amino acid is not Gly.

According to a preferred embodiment of the invention, TP consists in an uninterrupted stretch of 22 collagen repeats. More preferably, TP consists in the stretch of 22 collagen repeats of SEQ ID NO 1, corresponding to amino acids 45 to 110 of mACRP30, as represented in SEQ ID NO 2 of WO 96/39429:
**Gly** Ile **Pro** **Gly** His **Pro** **Gly** His Asn **Gly** Thr **Pro** **Gly** Arg Asp **Gly** Arg Asp **Gly** Thr **Pro Gly** Glu Lys **Gly** Glu Lys **Gly** Asp Ala **Gly** Leu Leu **Gly** Pro Lys **Gly** Glu Thr **Gly** Asp Val **Gly** Met Thr **Gly** Ala Glu **Gly** Pro Arg **Gly** Phe **Pro** **Gly** Thr **Pro** **Gly** Arg Lys **Gly** Glu **Pro** **Gly** Glu Ala

According to another preferred embodiment of the invention, TP consists in the stretch of 22 collagen repeats corresponding to amino acids 42 to 1107 of hACRP30, as represented in SEQ ID NO 7 of WO 96/39429:

DP are known in the art and comprises dimerization fragments of immunoglobulins (Fc fragments), the C-terminal dimerization domain of osteoprotegerin (Receptor: δN-OPG; amino acids 187-401), or polypeptides sequences comprising at least 6, preferably 8 to 30 amino acids and allowing dimerization. These peptides generally comprise at least a cysteine residue allowing the formation of disulfide bonds. Other polypeptides useful as DP according to the invention are peptides designated as "leucine zippers" comprising a Leucine residue being present every seventh residue.

Examples of such peptides comprising at least a cysteine residue comprise the following peptides:
Val Asp Leu Glu Gly Ser Thr Ser Asn Gly Arg Gln Cys Ala Gly Ile Arg Leu
Glu Asp Asp Val Thr Thr Thr Glu Glu Leu Ala Pro Ala Leu Val Pro Pro Pro Lys Gly Thr **Cys** Ala Gly Trp Met Ala
Gly His Asp Gln Glu Thr Thr Thr Gln Gly Pro Gly Val Leu Leu Pro Leu Pro Lys Gly Ala **Cys** Thr Gly Trp Met Ala.

The second sequence above corresponds to amino acids 17 to 44 of mACRP30 as represented in SEQ ID NO 2 of WO 96/39429, and the third sequence above corresponds to amino acids 15 to 41 of SEQ ID NO 7 of WO 96/39429.

Other peptides comprising at least one cysteine residue, can be found in amino acid sequences upstream the stretch of collagen repeats of molecules having a structure analogous to ACRP30 (ACRP30-like) as disclosed in WO 99/10492, WO 99/59618, WO 99/59619, WO 99/64629, WO 00/26363, WO 00/48625, WO 00/63376, WO 00/63377, WO 00/73446, WO 00/73448 or WO 01/32868.

Leucine zippers are well known in the art and can be found in natural proteins and eventually identified using bioinformatics tools available to the one skilled in the art (http://www.bioinf.man.ac.uk/zip/faq.shtml; http://2zip.molgen.mpg.de/; Hirst, J.D., Vieth, M., Skolnick, J. & Brooks, C.L. III, Predicting Leucine Zipper Structures from Sequence, Protein Engineering, 9, 657-662 (1996)).

The constitutive elements L, H, HP, TP and/or DP in the polypeptides of formula I, Ia, Ib or Ic, according to the invention, are assembled by peptides bonds. They may be separated by "linkers" who will not affect the functionality of the polypeptide according to the invention, its ability to form hexamers and to bind with the receptor corresponding to the ligand L. Such linkers are well known in the art of molecular biology.

The polypeptide according to the invention may also comprise peptide sequences on its N-terminus and/or C-terminus, which will not affect the functionality of the polypeptide according to the invention. These peptides may comprise affinity tags, for purification or detection of the polypeptide according to the invention. Such affinity tags are well known in the art and comprise a FLAG peptide (Hopp et al., Biotechnology 6: 1204 (1988)) or a Myc-His tag.

According to a preferred embodiment of the invention, H comprises a dimerization polypeptide (DP) and a trimerization polypeptide (TP), and is most preferably represented by the following formula:_DP-TP - L (Ib)_wherein R, DP and TP are defined above and below.

More preferably, DP and TP represent together amino acids 17 to 110 of mACRP30 as represented in SEQ ID NO 2 of WO 96/39429 or amino acids 15 to 107 of hACRP30 as represented in SEQ ID NO 7 of WO 96/39429.

As a preferred embodiment of the invention the polypeptide comprises the fusion polypeptide m or hACRP30:hFasL (MegafasL), more particularly m or hACRP30:hFasL disclosed in WO 01/49866.

According to another embodiment of the invention, the hexamerization moiety comprises a Fc portion of IgG comprising amino acids 248 to 473 of gi2765420, as disclosed in the PCT application No. PCT/EP02/09354.

In the method according to the invention, soluble multimerized Fas-L molecules can be used in combination with other known means of treatment. Such "means of treatment" comprise other molecules or compositions used in oncology. Such other molecules or compositions suitable are well known in the art, such as any of the molecules or compositions listed under the heading "Cancerologie" in the Dictionaire Vidal (2003 ed.), in the Merk Index or in the Physician Desk Reference, including doxorubicin, platinum salts like cisplatin and velcade.

The present invention also concerns a new in vitro method for preparation of harvested cells substantially devoid of malignant cells, comprising the step of treating a sample of harvested cells with a sufficient amount of soluble multimerized Fas-L molecules according to claim 2.

### 1. Test the cytotoxicity of MegaFasL on haematopoietic cancer cells and cell lines.

The aim is to determine the sensitivity of a panel of hematopoietic cancer cells and cell lines to MegaFasL. Cells and cell lines will include preferentially those originating from patients with multiple myeloma (MM), follicular lymphoma (FL), mantle cells lymphoma (MCL), chronic lymphocytic leukemia (CLL), diffuse large cell lymphoma (DLCL), and acute myeloid leukemia (AML). The selection criterion for choosing these cells is based on the current autologous grafting indication for treating such patients.

Fas expression is determined on a panel of cell lines and primary cells from patients by FACS. Sensitivity to Fas-mediated killing is measured in a dose response and time course analysis using a PMS/MTS assay. Apoptosis is followed by DNA fragmentation, as well as in TUNEL and annexin V assays by FACS.

### 2. Test the cytotoxicity of MegaFasL on PBPC and immune cells.

First, Fas expression on peripheral blood progenitor cells (PBPC) and immune cells is determined by FACS notably using CD34 as a marker for progenitor cells, and CD3 and CD20 as markers for immune T and B cells, respectively. Sensitivity to Fas-mediated killing is determined in a PMS/MTS assay using antibody coated magnetic beads purified populations of PBPC and immune cells. Alternatively, since there is a risk that purified PBPC behave differently to PBPC in a population of hematopoietic cells, the sensitivity of PBPC cells in the latter situation will also be investigated using multiple staining specific for apoptosis and cell lineage and analyzed by FACS. Apoptosis is followed by DNA fragmentation, as well as in TUNEL and annexin V assays by FACS. The sensitivity of PBPC and immune cells to Fas-mediated killing is compared to that of cancer cells.

Second, the capacity of PBPC treated with MegaFasL to generate pluripotent cells is determined *in vitro.* Both late and early progenitors are evidenced by colony-forming-unit and long-term initiating cell (LTC-IC) assays, respectively. If necessary, secondary CFU is determined, such assay evidence cells at earlier stage of differentiation than those evidenced by CFU. All these assays are available and are routinely performed in the laboratory. LTC-IC assay involves the determination of the number of colony forming unit (CFU) on semi-solid agarose gel containing differentiating factors after co-culture of PBPC on stromal cells. The integrity of immune (T) cells treated with MegaFasL is determined by measuring proliferation in an allogeneic reaction assay, and the production of specific thymus-dependent antibodies (such as those to tetanus toxoid). This latter capacity is evaluated on PBMC from donor boosted with tetanus toxoid one month prior to harvesting, and with such PBMC cultured in vitro in the presence of antigen for 14 days.

Finally, the engraftment capacity of PBPC treated with MegaFasL is tested *in vivo* in NOD/SCID mice. Human PBPC engraftment and expression is followed by: a) immunohistochemistry, FACS, and by PCR amplification of bone marrow, b) human CFU presence in BM cells. The integrity of immune cells towards antibody production will be determined after injection of human PBMC into SCID mice.

### 3. Test the cytotoxicity of MegaFasL on mixed populations of cancer cells and PBPC.

The sensitivity of hematopoietic cancer cells and PBPC to MegaFasL is tested *in vitro* as described above in mixed populations, using PCR to track cancer cells and monoclonal antibodies to track PBPC and immune cells. In addition, the integrity of PBPC treated with MegaFasL is determined in a LTC-IC assay. For *in vivo* studies, the engraftment capacity of PBPC and immune cells is tested using NOD/SCID and SCID mice, respectively. Readout will be the same as above. Mixtures of hematopoietic cancer cells and stem cells will be treated with MegaFasL or according to existing protocols. Treatment efficacy will by measured *in vitro* (LTC-IC assays) and *in vivo* (colonization in NOD/SCID mice).

### Description of the Figures

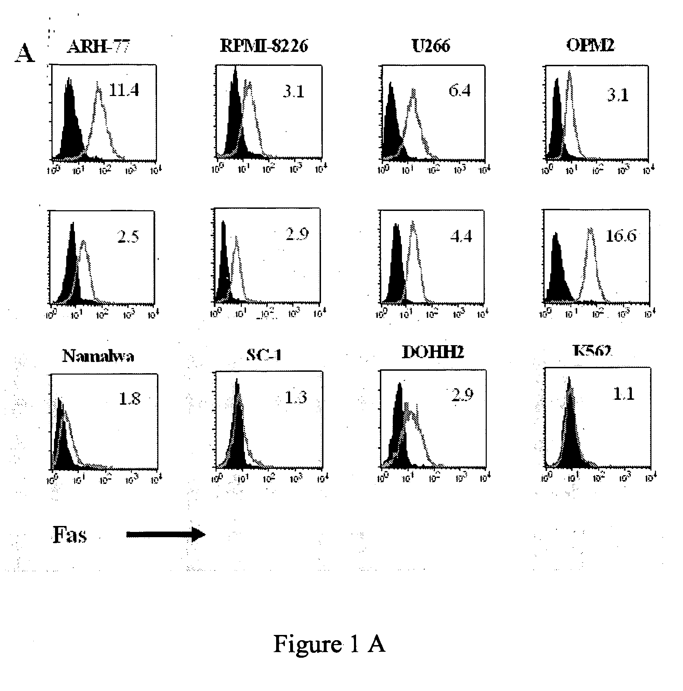

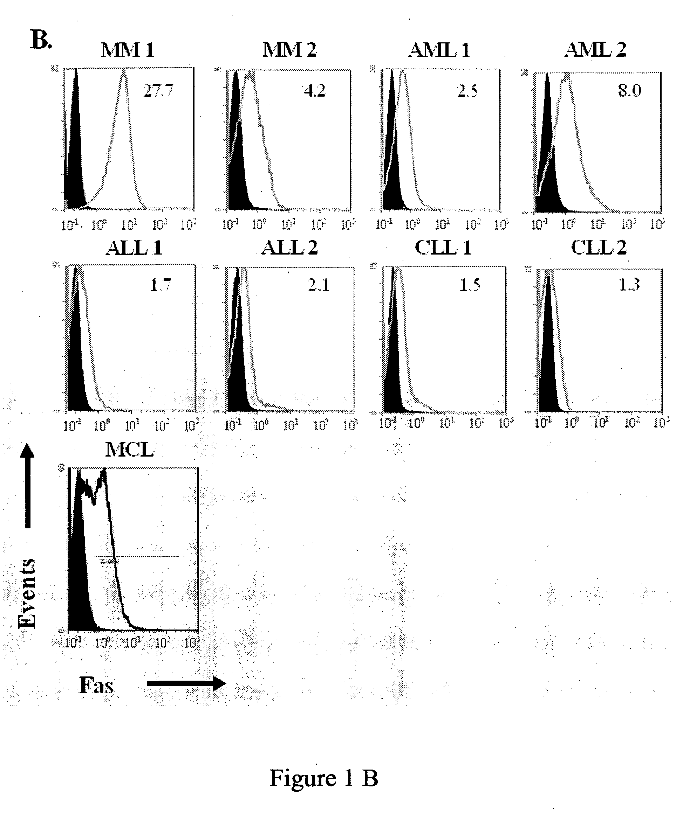

**Fig. 1****. Fas expression on haematopoietic cancer cells. (A.)** A panel of haematopoietic cancer cell lines were stained with a fluorochrome-labelled anti-human Fas antibody and assessed by FACS for Fas expression. The numbers represent the fold increase in mean fluorescence intensity compared to isotype control. **(B.)** As in (A.) except primary haematopoietic tumoural cells were analysed.

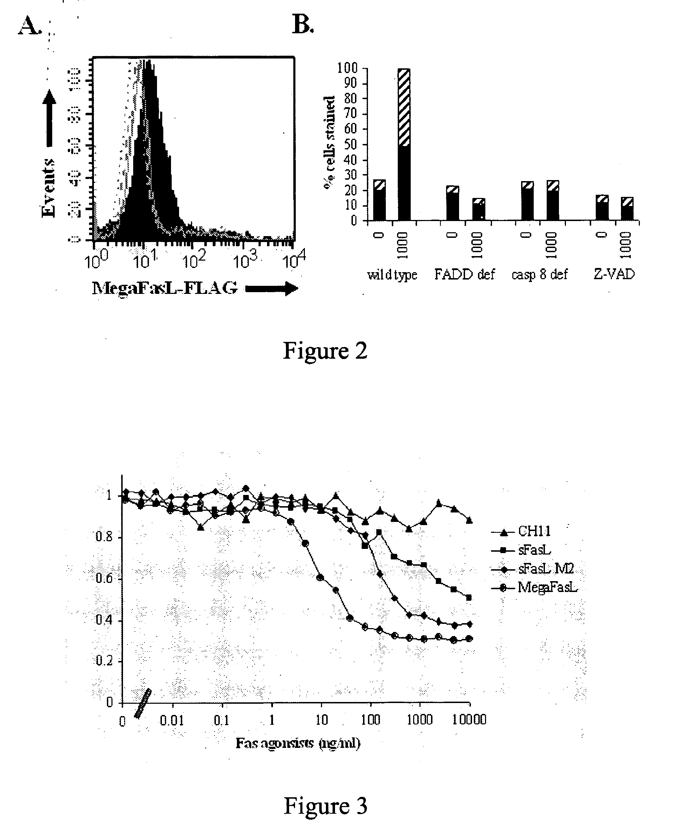

**Fig. 2****. MegaFasL specifically binds to Fas receptor and activates caspases. A.** ARH-77 cells were incubated with MegaFasL-FLAG (filled area), ZB4 and MegaFasL-FLAG (solid line), ZB4 (dashed line), or alone (dotted line). Cells were stained with a FITC-labelled monoclonal antibody against FLAG. **B.** Wild type Jurkat cells and those deficient in either FADD (FADD def) or caspase 8 (casp 8 def) were incubated for 5h with 1000ng/ml MegaFasL. Wild type Jurkat cells were also incubated for 5h with 1000ng/ml MegaFasL in the presence of Z-VAD (100 µM). Apoptosis was monitored by measuring annexin and 7AAD staining on cells using FACS. The percentage of annexin+ 7AAD+ cells are shown as solid black bars and that of annexin+ 7AAD- cells are shown as hatched bars. Data shown in A and B are representative of at least 3 independent experiments.
**Fig. 3****. PESMTS analysis illustrates that MegaFasL is a more potent killing agent than other Fas agonists.** The multiple myeloma cell line, ARH-77, was tested for their sensitivity to MegaFasL compared to other Fas agonists. Cells, cultured in medium containing 10% serum, were treated with Fas agonists for 15 hours then assessed for survival using the PESMTS assay. OD490 nm corresponds to survival.

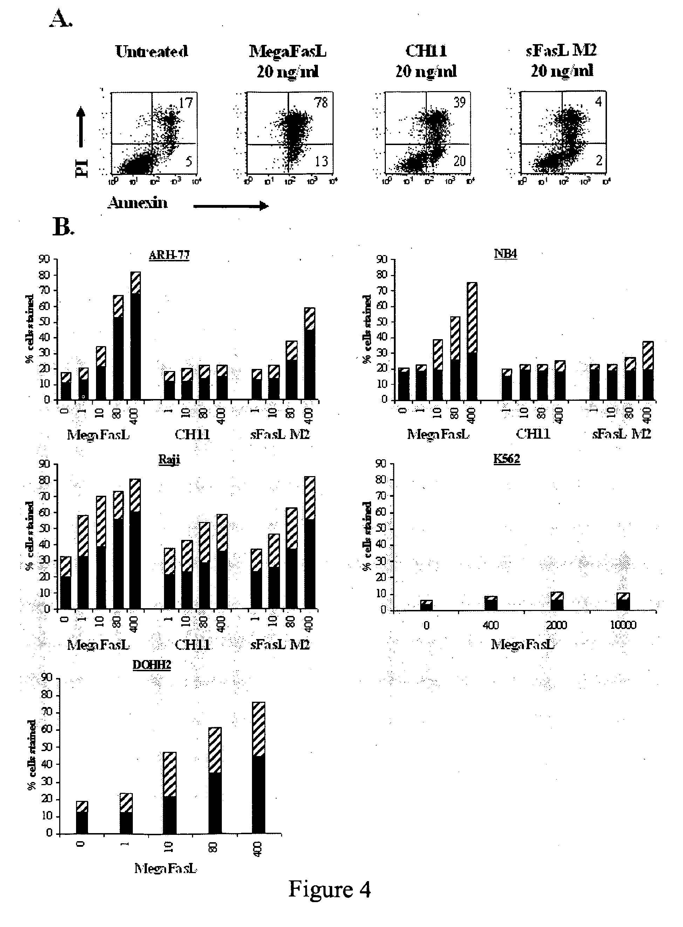

**Fig. 4****. Annexin V and PI staining of haematopoietic cancer cell lines treated with MegaFasL compared to other Fas agonists**. Cells were incubated with the indicated ng/ml MegaFasL, CH11 or sFasL M2 for 15 hours. Cells were stained with annexin and PI and analysed by FACS to determine the extent of apoptosis. **A.** represents an example of FACS data obtained on Jurkat cells and the numbers illustrate the percentage of cells that are annexin+ PI+ and annexin+ PI-. **B.** Graphical representation of MegaFasL, CH11 and sFasL M2 induced apoptosis in cell lines representing different haematological malignancies. Annexin+ PI+ (solid black bars) and annexin+ PI- (hatched bars).

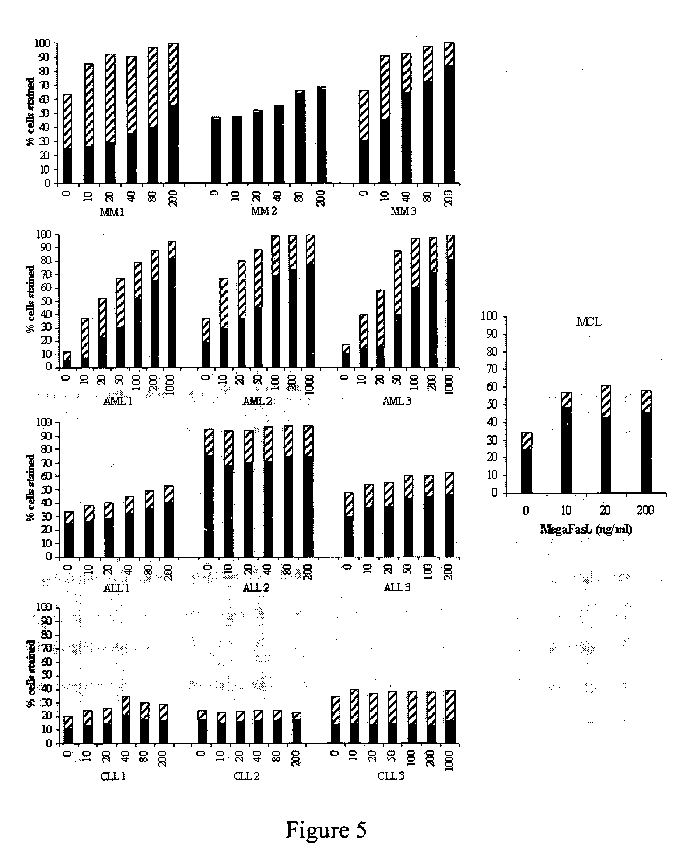

**Fig. 5****. Sensitivity of human haematological malignant primary cells towards MegaFasL**. Primary cells were incubated with various concentrations of MegaFasL. Apoptotic cells were determined by measuring annexin and 7AAD stained positive cells using FACS. Annexin+ 7AAD+ are shown as solid black bars and annexin+ 7AAD- are shown as hatched bars. MM, multiple myeloma; AML, acute myeloid leukaemia; ALL, acute lymphoblastic leukaemia; CLL, chronic lymphocytic leukaemia; MCL, mantle cell lymphoma.

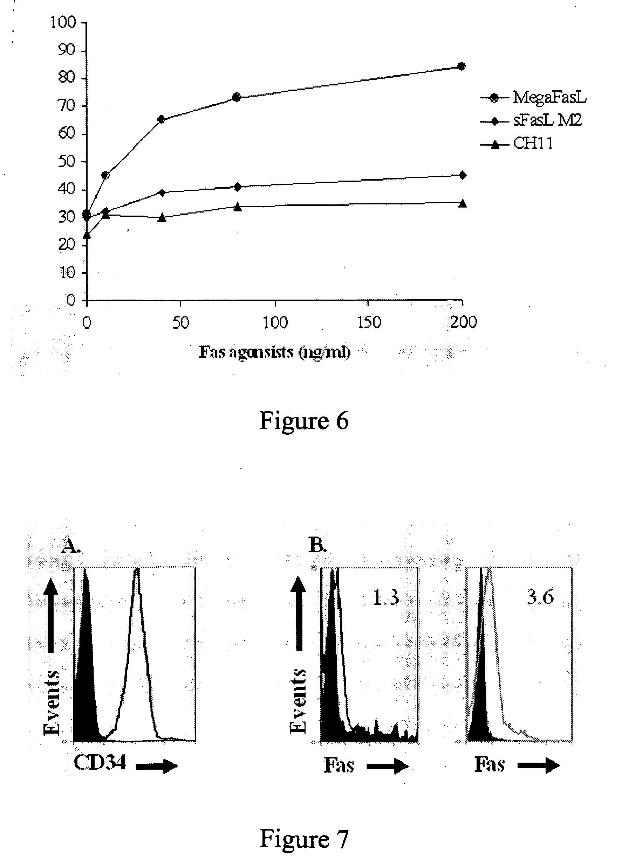

**Fig. 6****. Annexin V+/7AAD+ staining of primary multiple myeloma (IgA** λ**+, CD38+) cells treated with MegaFasL compared with other Fas agonists**. Cells were incubated with various concentrations (0, 10, 40, 80, 200 ng/ml) of MegaFasL, sFas M2 and CH11 for 5 hours. Percentage of cells with late apoptotic phenotype (annexin V+/7AAD+) was determined by FACS.
**Fig. 7****. CD34+ Haematopoietic stem cells express low levels of cell surface Fas. A.** A representative example of the haematopoietic stem cells used in the studies illustrating that the samples contain almost 100% percent CD34+ cells, as determined by FACS analysis. **B.** Illustrates the low, although differing, levels of cell surface Fas seen on CD34+ cells.

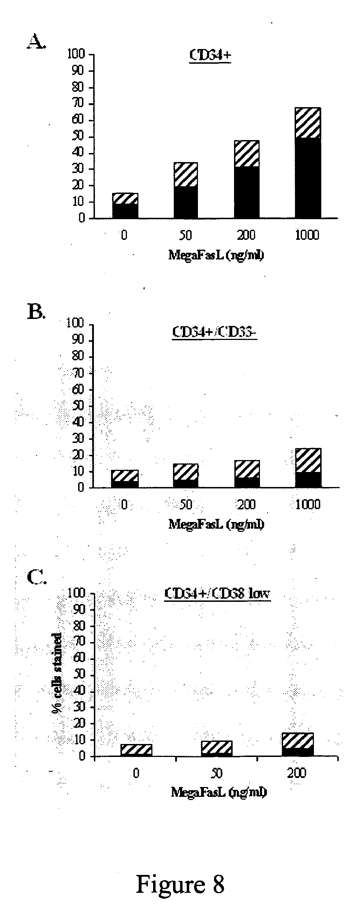

**Fig. 8****. Haematopoietic stem cells responsible for repopulating the haematopoietic system are resistant to MegaFasL. A.** Cells were incubated with the indicated concentrations of MegaFasL for 5 hours at 37°C. Cells were stained with an antibody against CD34 and CD34+ gated cells were anaylsed by FACS for levels of apoptosis (annexin-V and 7AAD staining). Annexin+ 7AAD+ are shown as solid black bars and annexin+ 7AAD- are shown as hatched bars. Representative example experiments are shown. **B and C.** As in A., except CD34+ CD33- and CD34+ CD38low cells were analysed for annexin and 7AAD staining. Representative experiments are shown.

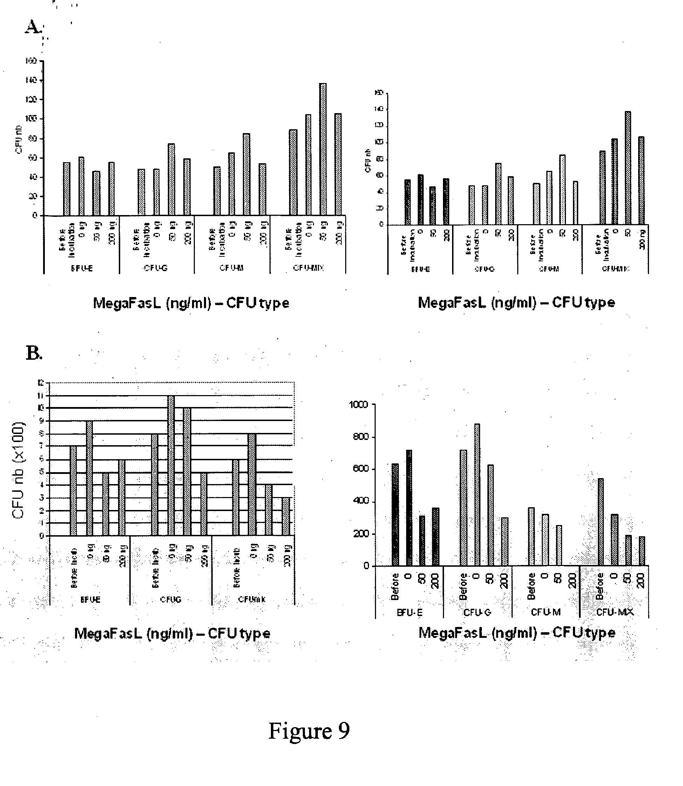

**Fig. 9****. Haematopoietic stem cells treated with MegaFasL are able to form colonies. A.** Haematopoietic stem cells were treated with the indicated concentrations of MegaFasL for 5 hours, washed and plated in CFU assays. Alternatively, haematopoietic stem cells were plated immediately after thawing (before-incubation). Colonies were counted after 2 weeks (CFU count). **B.** As in A., except cells were plated in LTC-IC assays and colonies were counted after 6 weeks. Representative experiments are shown. BFU-E, Burst-forming unit-Erythroid; CFU-G, Colony-forming unit-Granulocyte; CFU-M, Colony-forming unit-Macrophage.

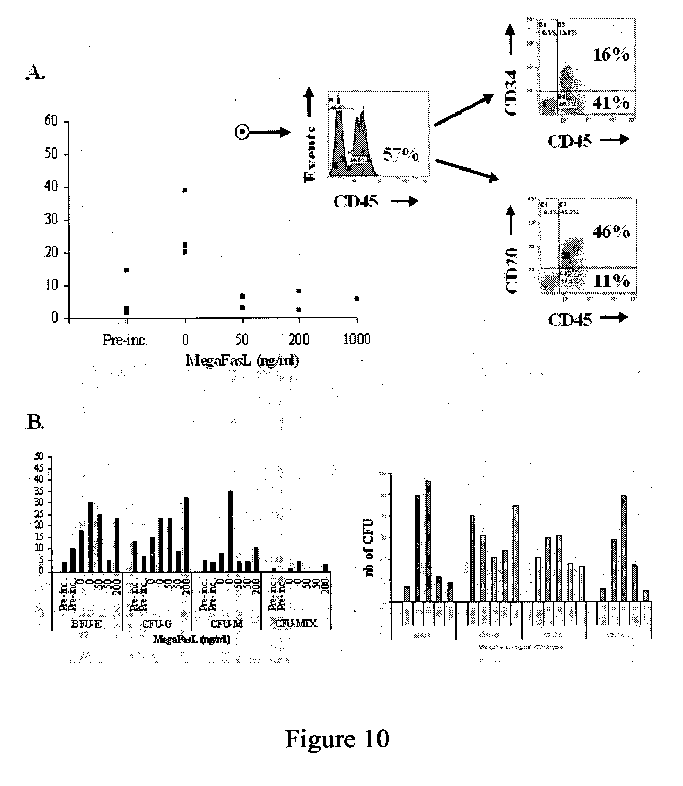

**Fig. 10****. Haematopoietic stem cells treated with MegaFasL have the capacity to engraft *in-vivo.* A.**CD34+ cells were exposed to MegaFasL for 5 hours, washed and transplanted into immunodeficient NOD/SCID mice. Alternatively, haematopoietic stem cells were plated immediately after thawing (pre-inc./before). After 6 weeks, mice were sacrificed and their marrow cells analysed by FACS for the presence of human CD45+ cells. O indicates the mouse in which a representative example of FACS analysis in shown, which illustrates the generation of different haematological lineages. **B.** Cells, from the marrow of NOD/SCID mice transplanted with CD34+ haematopoietic stem cells, were isolated after 6 weeks and 10000 cells were plated in CFU assays. Data shows 2 independent experiments. BFU-E, Burst-forming unit-Erythroid; CFU-G, Colony-forming unit-Granulocyte ; CFU-M, Colony-forming unit-Macrophage.

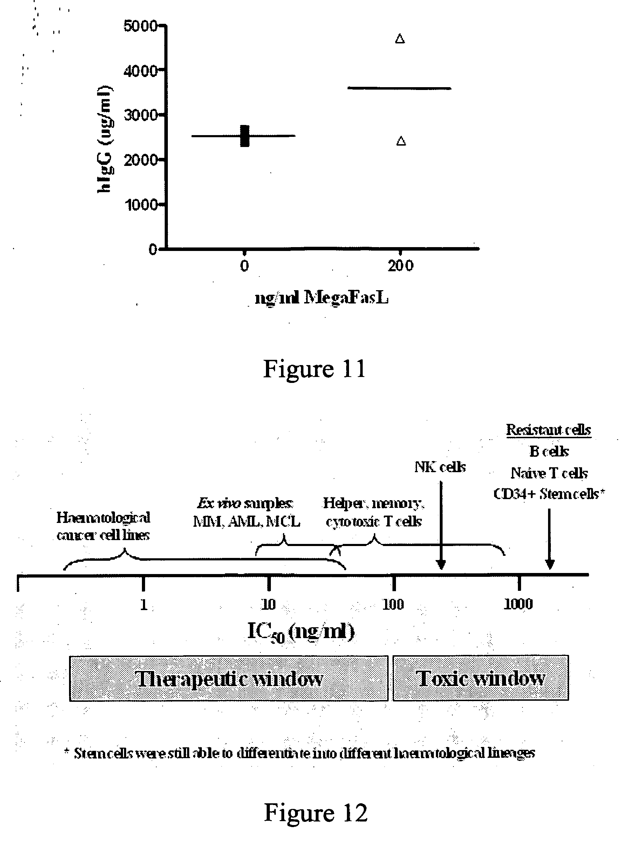

**Fig. 11****. Functional capacity of healthy immune cells treated with MegaFasL.** Peripheral blood mononuclear cells, obtained from whole blood by ficoll, were incubated with MegaFasL for 5 hours, washed and injected into SCID mice. After 36 days, the presence of human IgG in the mice sera was determined by FACS. This was interpreted as the capacity of the human immune cells treated with MegaFasL to engraft *in-vivo.* In addition, it remains feasible that tetanus toxoid may elicit an immune memory response from human PBMC incubated with MegaFasL (determined by the presence of anti-TT in mice sera). Experiments investigating this are currently ongoing. It is thought likely that using MegaFasL as an *ex-vivo* purging agent would only have a partial effect on the viability and function of differentiated immune cells.
**Figure 12****. Summary diagram showing sensitivity (IC₅₀) of different haematopoietic cells to MegaFasL.** The figure illustrates the therapeutic window in which MegaFasL would effectively kill tumour cells without affecting the capacity of haematopoietic stem cells to reconstitute an immune system, while having a partial effect on healthy immune cells in the graft. MM, multiple myeloma; AML, acute myeloid leukaemia; MCL, mantle cell lymphoma; NK, natural killer.

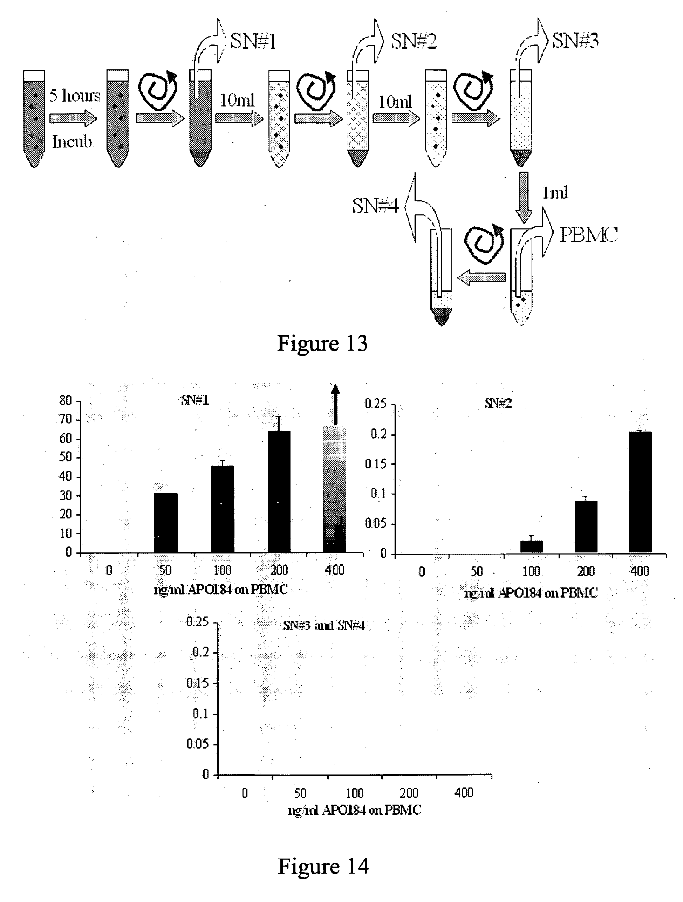

**Figure. 13****. Diagrammatic representation of the washing steps and collection of supernatants and PBMC for analysis**. PBMC were thawed and washed in RPMI 1640 medium containing 10% FBS and 1% penicillin/streptomycin (complete RPMI). PBMC were then resuspended at 10⁵/ml in complete RPMI and 10⁶ cells were incubated with 0, 50, 100, 200 or 400 ng/ml MegaFasL in polypropylene tubes for 5 hours at 37°C. The tubes were gently tapped every hour to prevent cells from adhering. At the end of the treatment, cells were centrifuged at 1100 rpm for 8 minutes and the supematent was collected (SN#1). The remaining supernatent was discarded and PBMC were resuspended in 10 ml of complete RPMI, centrifuged and the supematent was collected (SN#2). The procedure was repeated to obtain SN#3. PBMC were then resuspended in 1 ml complete RPMI and 100 µl (10⁵) PBMC were collected. The remaining 900 µl was centrifuged and the supematent was collected (SN#4).
**Figure. 14****. Washing removes detectable concentrations of MegaFasL (APO184).** PBMC were incubated with MegaFasL for 5 hours then supernatants (SN#1, SN#2, SN#3, SN#4) and PBMC from different stages of the washing procedure (see figure 13) were analysed by ELISA for the presence of MegaFasL. The change in colour of the 400 ng/ml bar in the SN#1 graph indicates that >80 ng/ml was present. In conclusion, two washes (SN#3) were sufficient to remove a detectable concentration of MegaFasL from the supernatent of centrifuged PBMC that had been incubated with MegaFasL for 5 hours.

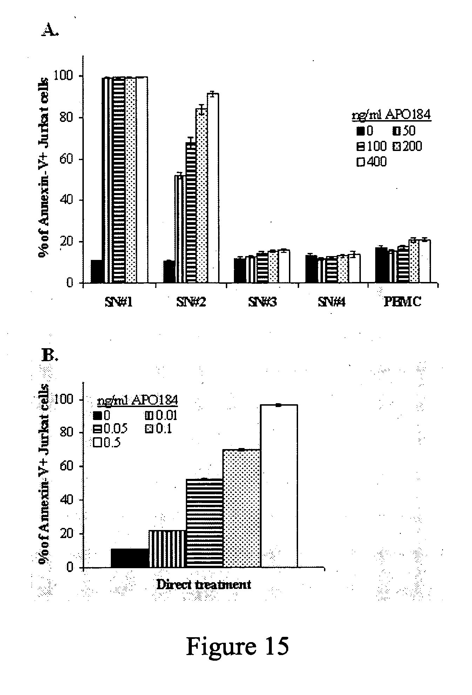

**Figure. 15****. A washing procedure removes active MegaFasL (APO184). A.** PBMC were incubated with MegaFasL for 5 hours then supernatants (SN#1, SN#2, SN#3, SN#4) and PBMC from different stages of the washing procedure (see figure 13) were incubated with Orange Cell Tracker stained Jurkat cells for 18 hours. Apoptotic Jurkat cells were determined by staining with annexin-V and analysing by flow cytometry. **B.** In parallel direct treatment of Jurkat cells with MegaFasL (0.01, 0.05, 0.1 or 0.5 ng/ml) for 18 hours illustrates the high sensitivity of Jurkat cells to MegaFasL. Indeed, 0.01 ng/ml MegaFasL increased apoptosis from 10.7 % to 21.8 %, a greater increase in apoptosis to that seen in Jurkat cells cultured with PBMC that had been incubated with 400 ng/ml then washed twice. Therefore, it can be deduced that less than 0.01 ng/ml MegaFasL was present in resuspended PBMC that had been washed twice after incubation with 400 ng/ml MegaFasL.

### MegaFasl induces high levels of apoptosis in haematological malignancies

IC₅₀'s determined from the PESMTS assay such as that represented in Fig. 3. Values represent the mean (±SEM) of 3 independent experiments. ND = no data obtained; Resistant = IC₅₀ was not reached at Fas agonist concentration up to 10,000 ng/ml.

**Table 1 - Comparison of haematopoietic cell lines sensitivity to different Fas agonists**

| **Disease** | **Cell name** | **MegaFasL IC₅₀ (ng/ml)** | **CH11 IC₅₀ (ng/ml)** | **sFasL M2 IC₅₀ (ng/ml)** |
|---|---|---|---|---|
| Multiple myeloma | ARH-77 | 10.4 ±0.19 | Resistant | 136 ±12.0 |
| | RPMI-8226 | 3.57 ±0.68 | Resistant | ND |
| | U266 | 6.20 ±1.50 | Resistant | 39.9 ±10.1 |
| | OPM2 | 2.19 ±1.53 | ND | ND |
| Acute myeloid leukaemia | HL60 (AML-M3) | 213 ±126 | Resistant | ND |
| | NB4 (AML-M3) | 16.0 ±3.18 | Resistant | Resistant |
| Acute T lymphoblastic leukemia | Jurkat | 0.06 ±0.03 | 6.58 ±2.57 | 6.36 ±1.93 |
| Burkitt's lymphoma | Raji (ALL-L3) | 5.60 ±2.80 | 15.8 ±0.06 | 12.1 ±2.07 |
| | Namalwa (ALL-L3) | 57.0 ±27.4 | Resistant | Resistant |
| Follicular lymphoma | SC-1 | Resistant | Resistant | Resistant |
| | DOHH2 | 1.17 ±0.41 | Resistant | Resistant |
| Chronic myeloid leukaemia | K562 | Resistant | Resistant | Resistant |

### Apoptotic effect of MegaFasL on immune cells

Peripheral blood mononuclear cells (PBMC) were stained with various markers to identify specific populations of immune cells and annexin-V to determine levels of apoptosis. The average percentage of annexin-V staining from 3 independent experiments are shown (±SEM). MegaFasL had an apoptotic effect on subtypes of PBMC, but importantly no specific immune cell type, except macrophages/monocytes/granulocytes, were completely eliminated (data not shown). Therefore, it remains feasible that immune cells incubated with MegaFasL could retain some functional capacity. Results are represented on Table 2.

**Table 2**

| **Immune cell type** | **Marker** | **0 ng/ml MegaFasL** | **10 ng/ml MegaFasL** | **200 ng/ml MegaFasL** |
|---|---|---|---|---|
| Leukocytes | CD45+ | 13.5 ±2.5 | 17.0 ±0.8 | 28.4 ±1.5 |
| T cell | CD3+ | 11.1 ±3.0 | 15.2 ±2.6 | 25.2 ±5.9 |
| Helper T cell | CD3+ CD4+ | 4.1 ±0.8 | 4.3 ±0.3 | 10.5 ±1.7 |
| Cytotoxic T cell | CD3+ CD8+ | 34.1 ±8.0 | 44.3 ±7.3 | 62.3 ±7.3 |
| Memory T cell | CD3+ CD45 RA-/RO+ | 4.2 ±0.5 | 9.3 ±1.4 | 46.8 ±10.0 |
| Naive T cell | CD3+ CD45 RA+/RO- | 3.4 ± 0.2 | 5.2 ±1.4 | 8.6 ±2.2 |
| B cells | CD19+ | 19.6 ±3.5 | 18.4 ±3.9 | 16.8 ±3.7 |
| Memory B cells | CD19+ CD27+ | 23.6 ±3.2 | 22.8 ±3.8 | 29.0 ±5.6 |
| Naive B cells | CD19+ CD27- | 18.4 ±4.7 | 17.1 ±5.4 | 14.6 ±4.4 |
| NK cells | CD2+CD3- | 13.7 ±5.1 | 15.1 ±4.7 | 39.9 ±2.0 |

### Method for elimination of malignant cells in the clinic

Currently in the clinic, HSC are obtained from the peripheral blood following growth factor injection, such as with granulocyte-colony stimulating factor (G-CSF) or granulocyte-macrophage colony stimulating factor (GM-CSF), which stimulates the movement of HSC from the bone marrow to the peripheral blood, a process called mobilization. The procedure performed to collect in a pouch the peripheral haematopoietic cells enriched in HSC is called leukapheresis. The resulting pouch of cells is then lowered to 4°C, prior to freezing in liquid nitrogen (-196°C). When the patient is ready for the transplant, cells are thawed at 37°C, washed to remove DMSO and transplanted into the patient immediately. MegaFasL will be incorporated into a clinical protocol between leukapheresis and reinfusion of cells into the patient.

## Claims

1. Method for ex-vivo purging of cells in autologous transplantation, wherein the sample of harvested cells from a patient with multiple myeloma (MM), follicular lymphoma (FL), mantle cells lymphoma (MCL), chronic lymphocytic leukaemia (CLL), diffuse large cell lymphoma (DLCL) or acute myeloid leukaemia (AML), is treated with a sufficient amount of a soluble multimerized form of Fas ligand which is a hexamer comprising six monomers, assembled together, each of the monomers comprising a polypeptide of formula (I):
H-L (I)
wherein
L comprises the extracellular domain of human FAS ligand (hFasL), comprising amino acids Glu 139 to Leu 281 of hFasL,
H represents an N-terminal hexamerization moiety,
to kill malignant cells without substantially affecting viability of cells to be transplanted.

2. Method for *in vitro* preparation of harvested cells substantially devoid of malignant cells, comprising the step of treating a sample of harvested cells from a patient with multiple myeloma (MM), follicular lymphoma (FL), mantle cells lymphoma (MCL), chronic lymphocytic leukaemia (CLL), diffuse large cell lymphoma (DLCL) or acute myeloid leukaemia (AML), with a sufficient amount of a soluble multimerized form of Fas ligand which is a hexamer comprising six monomers, assembled together, each of the monomers comprising a polypeptide of formula (I):
H-L (I)
wherein
L comprises the extracellular domain of human FAS ligand (hFasL), comprising amino acids GLu 139 to Leu 281 of hFasL,
H represents an N-teiminal hexamerization moiety,
to kill malignant cells without substantially affecting viability of cells to be transplanted.

3. The method as claimed in anyone of claims 1 or 2, wherein the amount of the soluble multimerized form of Fas ligand in the solution is comprised between 1 and 400 ng/ml.

4. The method as claimed in any one of claims 1 to 3, wherein harvested cells are treated for a period comprised between 1 and 12 hours.

5. The method as claimed in anyone of claims 1 to 4, wherein after treatment with the multimerized form of Fas ligand, remaining living cells are treated by extensive washing.

6. The method as claimed in anyone of claims 1 to 5, wherein H comprises a dimerization polypeptide (DP) and a trimerization polypeptide (TP) and is represented by the formula : DP-TP-L.

7. The method as claimed in claim 6, wherein DP and TP represent together amino acids 17 to 110 of mACRP30 or amino acids 15 to 107 of hACRP30.

8. The method as claimed in anyone of claims 1 to 7, wherein harvested cells are stem cells.

## Patentansprüche

1. Verfahren zur Ex-vivo-Spülung von Zellen für autologe Transplantationen, wobei die Probe von geernteten Zellen von einem Patienten mit multiplem Myelom (MM), follikulärem Lymphom (FL), Mantelzellenlymphom (MCL), chronischer lymphozytärer Leukämie (CLL), diffusem großzelligem B-Zell-Lymphom (DLCL) oder akuter myeloischer Leukämie (AML) mit einer ausreichenden Menge einer löslichen multimerisierten Form des Fas-Liganden behandelt wird, bei dem es sich um ein Hexamer mit sechs Monomeren handelt, die zusammengesetzt sind, wobei jedes der Monomere ein Polypeptid der Formel (I)
H-L (I)
umfasst, wobei
L die extrazelluläre Domäne des humanen FAS-Liganden (hFasL) umfasst, welche die Aminosäuren Glu 139 bis Leu 281 von hFasL umfasst,
H einen N-terminalen Hexamerisationsteil darstellt,
um maligne Zellen zu töten, ohne die Lebensfähigkeit zu von transplantierenden Zellen im Wesentlichen zu beeinträchtigen.

2. Verfahren zur In-vitro-Herstellung von geernteten Zellen, die im Wesentlichen frei von malignen Zellen sind, umfassend den Schritt des Behandelns einer Probe von geernteten Zellen von einem Patienten mit multiplem Myelom (MM), follikulärem Lymphom (FL), Mantelzellenlymphom (MCL), chronischer lymphozytärer Leukämie (CLL), diffusem großzelligem B-Zell-Lymphom (DLCL) oder akuter myeloischer Leukämie (AML) mit einer ausreichenden Menge einer löslichen multimerisierten Form des Fas-Liganden, bei dem es sich um ein Hexamer mit sechs Monomeren handelt, die zusammengesetzt sind, wobei jedes der Monomere ein Polypeptid der Formel (I)
H-L (I)
umfasst, wobei
L die extrazelluläre Domäne des humanen FAS-Liganden (hFasL) umfasst, welche die Aminosäuren Glu 139 bis Leu 281 von hFasL umfasst,
H einen N-terminalen Hexamerisationsteil darstellt,
um maligne Zellen zu töten, ohne die Lebensfähigkeit zu von transplantierenden Zellen im Wesentlichen zu beeinträchtigen.

3. Verfahren nach einem der Ansprüche 1 oder 2, wobei die Menge der löslichen multimerisierten Form des Fas-Liganden in der Lösung zwischen 1 und 400 ng/ml beträgt.

4. Verfahren nach einem der Ansprüche 1 bis 3, wobei die geernteten Zellen für eine Dauer von 1 bis 12 Stunden behandelt werden.

5. Verfahren nach einem der Ansprüche 1 bis 4, wobei nach der Behandlung mit der multimierisierten Form des Fas-Liganden die restlichen lebenden Zellen durch extensives Waschen behandelt werden.

6. Verfahren nach einem der Ansprüche 1 bis 5, wobei H ein Dimerisationspolypeptid (DP) und ein Trimerisationspolypeptid (TP) umfasst und durch die Formel DP-TP-L dargestellt ist.

7. Verfahren nach Anspruch 6, wobei DP und TP zusammen die Aminosäuren 17 bis 110 von mACRP30 oder die Aminosäuren 15 bis 107 von hACRP30 darstellen.

8. Verfahren nach einem der Ansprüche 1 bis 7, wobei es sich bei den geernteten Zellen um Stammzellen handelt.

## Revendications

1. Procédé d'élimination *ex vivo* de cellules dans une transplantation autologue, dans lequel l'échantillon des cellules récoltées à partir d'un patient souffrant d'un myélome multiple (MM), d'un lymphome folliculaire (LF), d'un lymphome à cellules du manteau (LCM), d'une leucémie lymphocytaire chronique (LLC), d'un lymphome diffus à grandes cellules (LDGC) ou d'une leucémie myéloïde aiguë (LMA), est traité avec une quantité suffisante d'une forme multimérisée soluble du ligand Fas qui est un hexamère comprenant six monomères, assemblés ensemble, chacun des monomères comprenant un polypeptide de formule (I) :
H-L (I)
dans laquelle
L comprend le domaine extracellulaire du ligand Fas humain (hFasL), comprenant les acides aminés Glu 139 à Leu 281 de hFasL,
H représente un radical d'hexamérisation N-terminal,
pour tuer les cellules malignes sans pratiquement affecter la viabilité des cellules à transplanter.

2. Procédé de préparation *in vitro* des cellules récoltées pratiquement dépourvues de cellules malignes, comprenant l'étape consistant à traiter un échantillon de cellules récoltées à partir d'un patient souffrant d'un myélome multiple (MM), d'un lymphome folliculaire (LF), d'un lymphome à cellules du manteau (LCM), d'une leucémie lymphocytaire chronique (LLC), d'un lymphome diffus à grandes cellules (LDGC) ou d'une leucémie myéloïde aiguë (LMA), avec une quantité suffisante d'une forme multimérisée soluble du ligand Fas qui est un hexamère comprenant six monomères, assemblés ensemble, chacun des monomères comprenant un polypeptide de formule (I) :
H-L (I)
dans laquelle L comprend le domaine extracellulaire du ligand Fas humain (hFasL), comprenant les acides aminés Glu 139 à Leu 281 de hFasL,
H représente un radical d'hexamérisation N-terminal,
pour tuer les cellules malignes sans pratiquement affecter la viabilité des cellules à transplanter.

3. Procédé selon l'une quelconque des revendications 1 ou 2, dans lequel la quantité de forme multimérisée soluble du ligand Fas dans la solution est comprise entre 1 et 400 ng/ml.

4. Procédé selon l'une quelconque des revendications 1 à 3, dans lequel les cellules récoltées sont traitées pendant une période comprise entre 1 et 12 heures.

5. Procédé selon l'une quelconque des revendications 1 à 4, dans lequel après traitement avec la forme multimérisée du ligand Fas, les cellules vivantes restantes sont traitées par un lavage intensif.

6. Procédé selon l'une quelconque des revendications 1 à 5, dans lequel H comprend un polypeptide de dimérisation (PD) et un polypeptide de trimérisation (PT) et est représenté par la formule : PD-PT-L.

7. Procédé selon la revendication 6, dans lequel PD et PT représentent ensemble les acides aminés 17 à 110 de mACRP30 ou les acides aminés 15 à 107 de hACRP30.

8. Procédé selon l'une quelconque des revendications 1 à 7, dans lequel les cellules récoltées sont des cellules souches.
